# EUROPEAN PATENT APPLICATION

(11) **EP 4 647 770 A1**
(43) Date of publication of application: **12.11.2025**
(21) Application number: 24174677.5
(22) Date of filing: 07.05.2024
(51) Int. Cl.: G01N 33/543, C07K 16/40, G01N 33/573

(54) **CONJUGATED ANTIBODIES AGAINST HUMAN MXA PROTEIN, KIT AND METHOD OF DETECTION OF HUMAN MXA PROTEIN**

(71) Applicant: BIOINOVA, A.S., 14200 Praha 4 (CZ)
(72) Inventor: Bauer, Peter, Praha 4 (CZ); Matoska, Vaclav, Praha 5 (CZ)
(74) Representative: Harber IP s.r.o.

(57) **Abstract**

The invention provides a pair of mouse monoclonal anti-human MxA protein antibodies, clones 1302.5.32 a 1302.34.16, wherein one of the antibodies is conjugated with biotin and the other of the antibodies is conjugated with detection particles.

Furthermore, the invention provides a lateral flow test kit for detection of human MxA protein in a tested sample, the kit comprising the pair of antibodies, lysis and reaction buffer, and a development device with an application zone for the application of the tested sample, a detection zone with a ligand of biotin and a control zone with anti-mouse antibody, wherein a membrane of the development device is based on nitrocellulose or cellulose acetate. The development device may directly include the antibodies in immobilized form.

## Description

### Field of Art

The present invention relates to a pair of conjugated antibodies against human MxA protein. Furthermore, the present invention relates to a kit for detection of human MxA protein comprising these antibodies and their use for detecting human MxA protein. The antibodies and the kit enable a very sensitive and rapid immunodetection of human MxA protein.

### Background Art

Unnecessary antibiotic treatment and overuse of antibiotics leads to an enormous increase in antibiotic resistance, which threatens majority of the population. The level of such misuse has been illustrated for example by Fleming-Dutra et al. (2016, doi: 10.1001/jama.2016.4151) where data on ambulatory care visits in 2010/11 in the USA reveal that in acute respiratory conditions per 1000 population, only 50% antibiotic prescriptions were estimated to be appropriate and other data show that the majority of antibiotic prescriptions occur in outpatient setting (>60%) (Suda et al., 2013, https://doi.org/10.1093/jac/dks445). A major role for the antibiotic missuse plays the aetiology of acute respiratory infections and subsequent insufficient differential diagnostics. It is estimated that 50-60% of community acquired pneumonias are of viral, approximately 30% combined viral-bacterial and only 15-20% bacterial aetiology (Nascimento-Carvalho et al., 2016, https://doi.org/10.1186/s12887-016-0645-3). According to the World Health Organization (WHO), in 2050, the most common cause of death may become infections untreatable by antibiotics, which would overtake cardiovascular and oncological diseases. However, due to the rapid increase in resistance in some parts of the world, this situation is anticipated even sooner. Unfortunately, the global trend of the evolution of antibiotic-resistant bacteria is almost irreversible, and the only possible action is to mitigate its effects. This effort includes, in particular, the correct use of antibiotics, to which this invention will greatly contribute by reliable and timely identification of patients whose disease has viral origin.

Mx proteins are intracellular proteins belonging to dynamin superfamily proteins forming a distinct subclass of large GTPases, which play a crucial role in immune reaction in organism. These proteins are induced by type I (interferon α and β) and type III (interferon λ) interferons and are useful indicators of the state of the organism induced by an increased level of interferons, e.g. in viral or autoimmune diseases or during treatment with type I (including interferon α, β, and -ω) and III (interferons λ1-4) interferons. In humans, indicators of this condition are, among others, human proteins MxA and MxB. MxA is detectable at high levels 2 to 4 hours after exposure of cells to type I interferons, and is produced, among others, by peripheral blood mononuclear cells. For this reason, the MxA protein is proposed as a diagnostic tool for the detection of conditions that lead to elevated levels of type I and III interferons.

In the prior art, monoclonal antibodies against the human MxA protein, as well as their conjugates enabling their detection, are known. Methods for the detection of human MxA protein with rather high sensitivity (detection limit up to 1 ng/mL) using these monoclonal antibodies and their conjugates are also known. The article "A whole blood immunoassay for the interferon-inducible human Mx protein" by H. Towbin et al. (1992; DOI: 10.1089/jir.1992.12.67) describes the production and testing of human anti-MxA monoclonal antibodies (clones 1302.5.32 and 1302.34.16) with a detection limit of 5 ng/ml. These clones form a sandwich immunocomplex with the MxA protein, with clone 1302.5.32 binding to the MxA epitope at position 430-662 and clone 1302.34.16 at position 1-429, counting from the N-terminus of the MxA protein. In this publication, conjugated monoclonal antibodies are described in more detail, specifically clone 1302.5.32 conjugated with paramagnetic particles for anchoring the monoclonal antibody and clone 1302.34.16 conjugated with biotin, or with dimethyl acridinium ester (DMAE) for chemiluminescence detection. Biotinylated clone 1302.34.16 is detected after incubation with avidin conjugated to alkaline phosphatase and detection after enzymatic reaction with p-nitrophenyl phosphate substrate at 405 nm. A disadvantage of conjugation of biotin and clone 1302.34.16 is a reduced sensitivity of the clone to the MxA protein. Clones 1301.51.6, 1301.90.85 and 1319.35.126 (epitope at position 1-429) are also described. These clones had higher values of dissociation constants during the analysis, and, in addition, clone 1319.35.126 reacted non-specifically with animal Mx proteins.

The article "Quantitation of interferon-induced Mx protein in whole blood lysates by an immunochemiluminuscent assay: elimination of protease activity of cell lysates in this" by S. K. Oh et al. (1994, DOI: 10.1016/0022-1759(94)90352-2) describes a method for the detection of human Mx protein in whole blood lysate samples with a detection limit of 20 ng/ml. It relies on a combination of two antibodies - an anchoring antibody (mouse monoclonal antibody conjugated with paramagnetic particles) and a detection antibody (mouse monoclonal antibody chemiluminescently labeled with DMAE). The indicated antibodies correspond to clones 1302.5.32 and 1302.34.16 from Towbin et al. (1994). The US patent 5863742 describes anti-MxA monoclonal antibodies against the human MxA protein and a method for their detection using an ELISA test. In particular, mouse clones 1302.5.32 (conjugation with paramagnetic particles for anchoring) and 1302.34.16 (conjugation with DMAE for chemiluminescence detection) are mentioned. The indicated antibodies correspond to clones 1302.5.32 and 1302.34.16 from Towbin et al. (1994). The patent description includes a method of processing the sample with MxA and proteases using a lysis agent (non-ionic detergent), a denaturing agent (SDS, urea or guanidine-HCl), heating to 50 to 60 °C for 1 to 30 min., then reaction with the aforementioned pair of monoclonal antibodies and detection of the immunocomplex (or specifically using a monoclonal antibody conjugated with DMAE).

Patent EP 0725081 B1 describes anti-MxA monoclonal antibodies against the human MxA protein and hybridomas producing these antibodies. In particular, the hybridoma lines KM1122 to KM1135 are mentioned, and the monoclonal antibody clones obtained from them (14 clones in total). The best results with regard to the specificity of the reaction and the response in cell assays (i.e. cells stimulated by means of interferon α or viral infection) were achieved by clones KM1124 and KM1135 (interferon α and viral infection) and clones KM1126 and KM1132 (interferon α only). Viral infections tested included influenza with an unspecified influenza virus, then viral diseases exanthema subitum (Human herpesvirus 6 or 7), hand, foot and mouth disease (Coxsackie virus A 16 or enterovirus 71), mumps (mumps virus), infectious mononucleosis (Epstein-Barr virus), pneumonia (it is not clear whether it is of viral origin) and chickenpox (Varicella zoster virus). Further analysis revealed binding sites on the MxA protein that show affinity to anti-MxA antibody epitopes - KM1126 and KM1135 (position 10-220), KM1124 (position 220-297) and KM1132 (position 468-662), counted from the N-terminus of the MxA protein. The disadvantage of these monoclonal antibodies is that said clones targeting the aforementioned epitopes provide a lower sensitivity of MxA protein detection than clones 1302.5.32 and 1302.34.16 from Towbin et al. (1994).

Patent application EP 1489416 A1 describes the use of the KM1135 anti-MxA monoclonal antibody clone and European patent application EP 2085782 A1 also describes the use of the KM1124-KM1135 anti-MxA monoclonal antibody clones.

The article "Evaluation of a combined MxA and CRP point-of-case immunoassay to identify viral and/or bacterial immune response in patients with acute febrile respiratory infection" by R. Sambursky et al. (2015, DOI: 10.3402/ecrj.v2.28245) describes a combined immunoassay for MxA and CRP proteins in acute febrile respiratory tract infections using the lateral flow test (LFT) and ELISA tests. The publication regarding an immunoassay for MxA protein refers to monoclonal antibody conjugates corresponding to clones KM1124-KM1135 of EP 0725081 B1 (Kyowa Medex Co., Ltd.).. The authors state MxA values above 40 ng/ml as relevant, but the real cut-off for the physiological level of MxA is approximately 12 to 15 ng/ml. The result is, in practice, insensitive to positive samples with low MxA levels and the procedure showed low sensitivity (e,g, Brendish et al., 2024, DOI: https://doi.org/10.1016/j.jinf.2023.11.003) and its disadvantage is provision of only qualitative results.

Patent application WO 03028582 A2 describes clone 1319.35.126 of an anti-MxA monoclonal antibody, which reacts non-specifically with animal Mx proteins as well. In the article "Interferon-induced human protein with homology to protein Mx of influenza virus-resistant mice" by Staeheli and Haller (1985, DOI: 10.1128/mcb.5.8.2150) the clone 2C12 anti-MxA monoclonal antibody with an unknown epitope and IgG1 class is described.

Patent application WO 9524500 describes a method of detecting human MxA protein by ELISA using two monoclonal conjugated antibodies (clone 1302.5.32 conjugated with paramagnetic particles and clone 1302.34.14 conjugated with DMAE) with a detection limit of 1 ng/ml.

However, all the above-mentioned methods with high sensitivity, e.g. ELISA using bioluminescence, require specific and expensive equipment and expensive consumables, which are not available in ordinary ambulances. These tests are therefore carried out in specialized laboratories, usually *en masse*, after collecting a sufficient number of samples to be placed in e.g. 96-well plates, which further delays the obtaining of results.

The demanding requirements disqualify these methods from use as a modem point-of-care testing (POCT) methods for direct bedside testing, in the outpatient clinics of general practitioners and paediatricians, as well as in departments where rapid testing of the MxA level is essential for making timely decisions regarding the diagnosis and treatment of patients. Moreover, rapid POCT without laboratory facilities is important in everyday healthcare services in various set-ups including outpatient practices, field practices, small laboratories or homecare. Another example of practical use is the testing of blood donors in transfusion stations, when the non-infectiousness of the material is typically tested only after collection. This represents an increase in both personnel and material costs, because in cases of positive detection of a viral infection, it is necessary to dispose of the already collected material as infectious. With the use of the POCT, it is possible to eliminate infected donors already before the blood collection.

POCT usually uses specifically designed test kits, disposable and single-use devices, and in some cases also small single-use analysers. However, these tools and methods generally do not show as high detection sensitivity as the more sophisticated laboratory methods. The currently available FebriDx lateral flow assay test for the detection of MxA has insufficient sensitivity (40 ng/ml MxA) for clinical use and it is not intended for quantification of the MxA levels. Another POCT solution for MxA has recently been revealed. It is based however on fluorescence immunoassay and therefore requires a specific detection equipment while not being suitable for self-testing. A solution that would enable both sufficiently sensitive and at the same time sufficiently fast, quantitative and low-cost POCT assay in medical facilities or during self-testing is currently not available.

### Disclosure of the Invention

The aim of the present invention is to provide a test in the POCT format which would enable a sufficiently sensitive detection limit and quantification limit for detection of human MxA protein in tested samples, in particular in samples of whole blood.

The invention achieves this aim by providing a combination of conjugated anti-MxA monoclonal antibodies and a lateral flow test (LFT) design that can be used for simple and rapid tests in daily clinical practice. The combination of antibodies according to the invention, and in particular in conjunction with the LFT design according to the invention, detects the human MxA protein with a sensitivity exceeding the sensitivity of methods known in the prior art.

A first aspect of the present invention is a pair of mouse monoclonal antibodies against the human MxA protein, clones 1302.5.32 and 1302.34.16, wherein one of the antibodies is conjugated with biotin and the other of the antibodies is conjugated with detection particles, preferably with gold particles.

Mouse monoclonal antibody 1302.5.32 against human MxA protein is obtainable from the hybridoma line "Hybridoma Mx 1302.5.32", accession number HB-11836 (ATCC).

Mouse monoclonal antibody 1302.34.16 against human MxA protein is obtainable from the hybridoma line "Hybridoma Mx 1302.34.16", accession number HB-11837 (ATCC).

In this aspect of the invetion, the invention includes the mouse monoclonal antibody 1302.5.32 against the human protein MxA, conjugated with biotin or detection particles. A further object of the present invention is the mouse monoclonal antibody 1302.34.16 against the human protein MxA, which is conjugated with biotin or detection particles.

The molar ratio of individual components in the conjugation reaction of the antibody with biotin ranges from 1:33 to 1:7 (antibody:biotin), with a ratio of 1:8 being the most preferrred, and a suitable biotinylation agent can be sulfo-N-hydroxysuccinimide-aminocaproyl-aminocaproyl-biotin (sulfo-NHS-LC-LC-biotin). Other suitable biotinylating agents may be, for example, NHS-Biotin, NHS-Desthiobiotin, Sulfo-NHS-Biotin, NHS-PEG12-Biotin, NHS-LC-Biotin, Sulfo-NHS-LC-Desthiobiotin, Sulfo-NHS-LC-Biotin, NHS-LC-LC-Biotin, NHS-SS-Biotin, Sulfo-NHS-SS-Biotin, NHS-PEG4-Biotin, NHS-SS-PEG4-Biotin, PFP-Biotin (described e.g. here: https://www.thermofisher.com/cz/en/home/life-science/protein-biology/protein-labeling-crosslinking/biotinylation.html). In many standard conjugation methods, the affinity of the antibody to the antigen is reduced due to conjugate interference. However, conjugation of the antibody with biotin under the above conditions surprisingly does not change the affinity of the antibody to the target epitopes on the MxA protein, and therefore even the antibody conjugated in this way targets the C-terminal epitope of the human MxA protein, especially position 430-662 in the case of antibody 1302.5.32, or to the N-terminal epitope of the human MxA protein, particularly position 1-429, in the case of antibody 1302.34.16, respectively.

Detection particles are in particular gold particles. Alternatively, dyed latex particles or cellulose nanospheres can also be detection particles.

Gold particles can be, for example, gold nanoparticles or colloidal gold particles, wherein several antibody molecules are conjugated on the surface of one gold particle. Said conjugation with gold particles (or with latex particles or cellulose nanospheres) does not change the affinity of the antibody to the target epitopes on the MxA protein, and therefore the conjugated antibody also targets the C-terminal epitope of the human MxA protein, especially position 430-662 in the case of antibody 1302.5.32, or to the N-terminal epitope of the human MxA protein, particularly position 1-429, in the case of antibody 1302.34.16, respectively.

Conjugation of the antibody with gold particles, or with other detection particles, preferably takes place at pH 7 to 8.5, and more preferably at pH 8. During conjugation, for the sensitivity of the reaction, it is advantageous to use a blocking agent, for example bovine serum albumin, or more preferably casein.

In a second aspect, the object of the present invention is a test device based on the principle of lateral flow test (LFT), which in combination with antibodies according to the invention leads to a synergistic increase in the specificity and sensitivity of the outcome.

In a first embodiment, object of this invention is a kit for the detection of the human MxA protein, which includes the pair of conjugated mouse monoclonal antibodies according to the invention against the human MxA protein, and a development device containing an application zone for the application of a tested sample, a detection zone with a ligand of biotin and a control zone with anti-mouse antibody. Conjugated mouse monoclonal antibodies are located separately from the development device, for example, they may be in a separate container or containers. In this embodiment, the conjugated antibodies and the human MxA protein interact before the tested sample is applied to the application zone of the development device. Conjugated mouse monoclonal antibodies should also be maintained separately from each other, i.e. placed in two separate compartments of one container or in two containers. If the conjugated anti-human MxA monoclonal antibodies were not separated during storage, they would react with each other and their interaction could cause a false positive signal on the test strip.

In a second embodiment, object of this invention is a kit for the detection of the human MxA protein, which includes a development device containing an application zone for the application of a tested sample, an interaction zone including separate spaces for immobilizing each of the pair of conjugated mouse monoclonal antibodies according to the invention against the human MxA protein, a detection zone with a ligand of biotin and a control zone with anti-mouse antibody. In this embodiment, the said conjugated antibodies and the human MxA protein interact only after the application of the tested sample to the application zone and subsequent migration of the tested sample from the application zone to the interaction zone of the development device. Antibodies of the invention can be immobilized in the interaction zone by applying them into their respective spaces and drying, or for example by reversible chemical bonding with a carrier in the interaction zone.

If the conjugated monoclonal antibodies against human MxA were not separated in the interaction zone, e.g. if they were applied to one common pad, then they would react together, and their mutual interaction could thus give rise to a false positive signal on the test strip. Therefore, each antibody is placed separately in separate spaces of the interaction zone, e.g. on separate pads placed behind each other.

Before being used in the kit for the detection of human MxA protein, the antibody conjugated with detection particles, preferably with gold particles, is preferably subjected to sonication (exposure to ultrasound) - this step significantly reduces its aggregation and improves the outcome.

The development device is formed on a membrane. For the development device, a PVDF (polyvinylidene fluoride) membrane is typically used in the prior art, because PVDF membrane exhibits a relatively high sensitivity. However, its disadvantage is its non-specificity and the fact that it causes a higher background. For the purposes of effective detection of the human MxA protein in the kit according to the present invention, we propose the use of a membrane based on nitrocellulose or cellulose acetate, which generates a lower signal, but is specific, so that in combination with the antibodies of the invention it is possible to achieve a sufficiently high sensitivity.

Preferably, in both embodiments of the human MxA protein detection kits, the biotin ligand in the detection zone is streptavidin. To achieve high sensitivity of the test, a streptavidin solution with a concentration of 0.8 mg/ml to 1.5 mg/ml is preferably used for preparing the development device. The streptavidin solution is applied to the detection zone in an amount of 0.75 µL/cm per width of 0.2 mm. The concentration of streptavidin in the detection zone is therefore 0.6 to 1.125 µg/cm². A streptavidin concentration of 0.8 mg/mL to 1.2 mg/mL is more preferred, and 1.2 mg/mL is most preferred concentration (resulting in concentration in the detection zone 0.9 µg/cm², total amount 72 ng/LFT). A lower concentration of streptavidin would lead to a decrease in sensitivity, and higher concentrations do not have an additive effect but they can cause the presence of a false positive signal.

Preferably, in both embodiments of the human MxA protein detection kits, the anti-mouse antibody in the control zone is anti-mouse IgG. The anti-mouse IgG solution is preferably used at a standard concentration of 1 mg/ml per area of 4 x 0.2 mm.

In both embodiments, the resulting sandwich immunocomplex is captured in the detection zone via the biotin-ligand (preferably biotin-streptavidin) bond. In the presence of human MxA protein in the tested sample, a sandwich immunocomplex of MxA protein with both conjugated mouse monoclonal antibodies is formed, which binds in the detection zone via biotin binding to the ligand of biotin. The remaining unreacted biotin-conjugated mouse monoclonal antibody also binds in the detection zone, and the remaining unreacted mouse monoclonal antibody conjugated to the detection particles binds in the control zone through binding of the mouse antibody to anti-mouse IgG. The visual signal is provided by the detection particles.

In the presence of unreacted antibodies (i.e., in the absence of human MxA protein in the tested sample applied to the application zone), capture of unreacted mouse monoclonal antibody conjugated to biotin in the detection zone will occur via biotin-streptavidin binding, and capture of unreacted mouse monoclonal antibody conjugated to detection particles in the control zone via binding of mouse antibody and anti-mouse IgG.

The detection occurs on the basis of the presence of an antibody conjugated with detection particles, either only in the control zone (negative test result, only test functionality check), or in both the detection and control zone (positive test result, presence of the sandwich immunocomplex and test functionality check). In the absence of signal in the control zone, the test becomes invalid because it appears that it did not function properly or that some of the key components failed.

A waste collection zone may be provided behind the control zone to capture all other unreacted components.

Preferably, any one of the two embodiments of the kit for detecting the human MxA protein further includes at least one lysis and reaction buffer. The lysis and reaction buffer may include at least one of the following components: TRIS (tris(hydroxymethyl)aminomethane), NaCl, KCl, NaH₂PO₄, KH₂PO₄, Tween 20 (polysorbate 20), BSA (bovine serum albumin), RPLA1 (50S ribosomal protein L1), casein hydrolysate, NaN₃, CHAPS (3-[(3-cholamidopropyl)dimethylammonio]-1-propanesulfonate), N,N-diethanolamide, Block Ace (blocking agent with bovine milk proteins), HEPES, Brij L23 or ELISA SynBlock. The lysis and reaction buffer preferably has a pH between 7 and 9, more preferably between 7.5 and 8.5, e.g. 7.5, 8 or 8.5, and most preferably a pH of 8.5.

In an even more preferred embodiment, the sensitivity of the kit for detecting the human MxA protein according to the invention is increased by the choice of lysis and reaction buffer. The choice of different buffers is large, while high sensitivity and the absence of false positive signals are facilitated by a low concentration of NaCl (below 200 mM) and a higher concentration of albumin (from 1 to 3% w/v BSA), or 0.3-1% w/v of casein hydrolyyate or 1-4% w/v RPLA1, as well as using CHAPS detergent. Tween 20 and some other detergents can lead to reduced sensitivity.

A particularly preferred embodiment of the lysis and reaction buffer, which leads to particularly good sensitivity and assists in decreasing the hemoglobin background and increasing the affinity of the anti-MxA antibodies, contains the following components:
- Tris-HCl in an amount within the range 75-125 mM
- NaCl or KCl in an amount within the range 100-1500 mM
- NaN₃ in an amount within the range 0,08-1.5% w/v
- CHAPS in an amount within the range 2-3% w/v
- N,N-Diethanolamid in an amount within the range 1-1.5% w/v
- 2-3% w/v BSA and/or 0.3-1% w/v casein hydrolysate and/or 1-4% w/v RPLA1.

An especially preferred composition of the lysis and reaction buffer contains 100 mM Tris-HCl, 150 mM NaCl, 0.1% w/v BSA, 0.1% w/v NaN₃, 2.5% w/v CHAPS, 1.2% w/v N,N-diethanolamide and 3% w/v RPLA1 or 0.5% w/v casein hydrolysate.

A most preferred composition of the lysis and reaction buffer contains 100 mM Tris-HCl, 150 mM NaCl, 0.1% w/v NaN₃, 2.5% w/v CHAPS, 1.2% w/v N,N-diethanolamide and 3% w/v BSA.

Testing has shown that the combination of mouse monoclonal antibody 1302.5.32 conjugated with biotin and mouse monoclonal antibody 1302.34.16 conjugated with detection particles, preferably with gold particles, works better when reacting with the human MxA protein in the lysis buffer, i.e. in the design of the kit according to the first embodiment where the immunoreaction of human MxA protein with conjugated mouse monoclonal antibodies occurs in solution, and only then the tested sample (with the antibodies) is applied to the application zone of the development device.

For the second embodiment of the kit according to the invention, where the antibodies are immobilized in the interaction zone of the development device, any of the mouse monoclonal antibodies 1302.5.32 and 1302.34.16 can be conjugated with detection particles, preferably with gold particles, and the other antibody can be conjugated with biotin. After the antibodies are applied to the development device, drying preferably takes place at room temperature for several hours, and drying of the biotin-conjugated antibody preferably takes place at a pH of 9 to 10 (the pH is ensured by a drying buffer). Antibody aggregation occurs at lower pH values.

A buffer containing NaCl, KH₂PO₄, Na₂HPO₄, KCl, with a pH within the range of 6.5 to 7.5, can be used for applying conjugated antibodies to the interaction zone of the system. Preferably, the buffer contains 137 mM NaCl, 1.8 mM KH₂PO₄, 10 mM Na₂HPO₄, 2.7 mM KCl with a pH of 6.8.

More preferably, to eliminate false positive signals, TRIS buffer (40 mM TRIS, 10% w/v trehalose (or alternatively sucrose), 0.1% w/v BSA, 0.25% w/v Tween, 0.05% w/v NaN₃, pH 9-10) is used to load the conjugated antibodies into the interaction zone of the system. Most preferably, the TRIS buffer described above with a pH of 10 is used.

Conjugated antibodies are preferably applied to the pads in the interaction zone at a concentration of 0.1-0.6 µg of biotinylated antibody per test and 50-80 mOD (2 µg of antibody per OD, therefore 0.1-0.16 µg of antibody per test) antibodies conjugated with detection particles (per pad size 5x6 mm). More preferably, 0.2 µg of biotinylated antibody and 50 mOD (0.1 µg) of antibody conjugated with detection particles are applied.

In the interaction zone of the test according to the second embodiment, pads are inserted, namely a sample pad (SP), a biotin conjugate pad (BCP) and a detection conjugate pad (DCP). The width of the pads is preferably 4 mm. The sample pad (SP) preferably has the length of 13-18 mm, more preferably 13-15 mm, most preferably 15 mm. The BCP and DCP pads are preferably 4-6 mm long, more preferably 5-6 mm, most preferably 6 mm.

The pads may be arranged in the order (in the direction from the application zone to the detection zone): SP-BCP-DCP or SP-DCP-BCP, more preferably SP-DCP-BCP.

The pads can be placed with an overlap which is preferably 1-4 mm, more preferably 2-3 mm, most preferably 3 mm.

It is advantageous if, in the kit according to the second embodiment of the invention, for the detection of the human MxA protein, the antibodies are applied in the following order in the direction from the application zone towards the detection zone: an antibody conjugated with detection particles and an antibody conjugated with biotin.

Another aspect of this invention is a method for detecting human MxA protein in a tested sample using the kit according to the invention, the method comprising the following steps:
a. lysis of capillary or venous whole blood sample in lysis and reaction buffer
b. in the presence of human MxA protein in the tested sample, forming a sandwich immunocomplex of the human MxA protein, biotin-conjugated mouse monoclonal antibody, and detection particle-conjugated mouse monoclonal antibody;
c. in the presence of human MxA protein in the tested sample, capturing the sandwich immunocomplex in the detection zone via binding of the biotin-conjugated mouse monoclonal antibody to the biotin ligand;
d. in the presence of human MxA protein in the tested sample, detecting the sandwich immunocomplex in the detection zone by means of a mouse monoclonal antibody conjugated to the detection particles in the detection zone, preferably visually, photometrically or densitometrically in the visible light spectrum; and
e. detecting the mouse monoclonal antibody conjugated with detection particles in the control zone, preferably visually, photometrically or densitometrically in the visible light spectrum.

The flow of the tested sample, conjugated antibodies and immunocomplex through the kit is preferably ensured by the addition of lysis and reaction buffer or by applying the tested sample in lysis and reaction buffer.

In one preferred embodiment, the first embodiment of the kit according to the invention is used, and the sandwich immunocomplex is obtained (if human MxA protein is present in the sample) by adding both conjugated mouse monoclonal antibodies to the lysed tested sample and by reacting them with the human MxA protein contained in the sample. Both the lysed sample and antibodies are typically used in lysis and reaction buffer. The thus modified tested sample is then applied to the application zone of the development device and detection is performed as described above.

In this embodiment, mouse monoclonal antibody 1302.5.32 is preferably conjugated to biotin and mouse monoclonal antibody 1302.34.16 is preferably conjugated to detection particles.

This procedure is particularly suitable for the detection of MxA, e.g. in clinics, laboratories or research institutes, where detection can be performed using a lateral test containing only a strip with streptavidin and anti-mouse IgG.

In another preferred embodiment, the second embodiment of the kit according to the invention is used, and the sandwich immunocomplex is obtained by applying the lysed tested sample to the application zone of the development device and the subsequent reaction of conjugated mouse monoclonal antibodies with the human MxA protein contained in the tested sample in the interaction zone of the development device. Preferably, the lysed tested sample is applied to the application zone in the lysis and reaction buffer, the sample then flows through the interaction zone containing conjugated antibodies, where the buffer provides the reaction environment, and the flow through the development device continues with the formed immunocomplex.

In both embodiments, after 2-10 minutes (preferably after 3-5 minutes, most preferably after 4 minutes) after the application of the lysed tested sample or of the mixture of lysed tested sample and antibodies to the application zone, an additional amount of lysis and reaction buffer is preferably added, which makes the flow of the sample through the LFT more efficient and at the same time, it washes away hemoglobin residues from lysed erythrocytes from the membrane and increases the detectability (sensitivity) of the test.

The second embodiment is also suitable for MxA detection, e.g. in households or in field conditions from a purchased kit. Both the procedure and the kit are manageable even for lay users. In a professional environment, in this embodiment, protein levels can be quantified with a dedicated reader (such readers are commercially available), in layman's use protein levels can be quantified using a dedicated mobile application.

Preferably, the tested sample is whole venous blood or whole capillary blood. Preferably, the lysed tested sample is a cell lysate of whole venous or capillary blood. The blood sample is introduced into the lysis and reaction buffer in which the cells are lysed, which ensures the detectability of the human MxA protein after its induction in the cells in the presence of type I and III interferons in peripheral blood mononuclear cells.

Another aspect of this invention is the use of the pair of mouse monoclonal antibodies 1302.5.32 and 1302.34.16, one of which is conjugated with biotin and the other is conjugated with detection particles, especially gold particles, for the detection of human MxA protein, and/or for the detection of viral or autoimmune disease, and/or for monitoring the response to treatment of viral or autoimmune diseases using interferon α and/or β, and/or in obscure infections in oncology patients. Viral diseases detectable by the presence of the human MxA protein include, for example, influenza virus infections, respiratory syncytial virus infections, parainfluenza virus infections, herpes simplex virus infections, Epstein-Barr virus infections, cytomegalovirus infections, adenovirus infections, coronavirus infections, rhinovirus infections, metapneumovirus infections, HIV infections, hepatitis B and C virus infections. Therapies of viral or autoimmune diseases using interferons include the therapy of e.g. hepatitis B and C or multiple sclerosis or certain types of tumors (e.g. lymphomas, melanoma, Kaposi's sarcoma, etc.).

The herein described invention provides a simple MxA protein detection system usable in clinical daily practice instead of applying extremely sensitive methods requiring special and expensive equipment to detect the amplified signal, e.g. chemiluminescence signal detection devices, ELISA reader, spectrometer, etc., and expensive consumables. Within the framework of this invention, suitable conditions and parameters for performing this rapid test were found, including reduction of the detection limit of antibodies by a specific combination of their conjugation, test kit design, preparation of analysed blood samples and suitable conditions for successful rapid test performance. In the present invention, no specific laboratory equipment is necessary for signal detection, as it is visible with the naked eye and quantifiable with simple optical density readers, which are a common part of the equipment of ambulances and various clinical departments, or mobile applications developed for optical density reading.

For human MxA protein detection, a population analysis established a cut-off value of 12 ng/ml whole blood (or interval 10 to 15 ng/ml) in healthy subjects. This means that 95% of "healthy" people have an MxA level below this value. Unfortunately, POCT methods known from the state of the art are only able to detect MxA levels 2-3x higher. The invention is able to detect 0.3 ng/ml of MxA protein in a sample, its sensitivity is therefore comparable to professional laboratory methods, which enables a precise analysis of the level of this protein in pathological conditions as well as determining its individual basal value. Fluctuations can then be evaluated from the point of view of personalized medicine and not only population medicine. The advantage of the present invention is therefore a sufficiently sensitive detection limit of the human MxA protein for the needs of reliable POCT testing or self-testing.

### Brief description of drawings

Fig. 1 shows the results of testing of different compositions of lysis and reaction buffers according to Example 1.
Fig. 2 shows the development device described in Example 9, with an application, interaction, detection, control and waste collection zone.
Fig. 3 shows a calibration curve generated by measuring different concentrations of purified native human MxA protein (Biovendor - Laboratorni medicína a.s., Cat. no. C1063111) in lysis and reaction buffer. Values on the Y-axis are Back Calculated Concentration (BCC). (Example 10)
Fig. 4 shows a sensitivity test using low levels of MxA; the calculated limit of detection is 0.3 ng/mL MxA. (Example 10)
Fig. 5 demonstrates LFT assays (embodiment with conjugated antibodies on pads) performed on 65 patient blood samples with different concentrations of MxA protein in whole blood (lowest level 0.9 ng/mL; highest level 650 ng/mL). (Example 10)
Fig. 6 shows the result of the comparison of the sensitivity of the antibodies according to the invention and the antibodies used in the FebriDx test. (Example 12)
Fig. 7 shows a comparison of the sensitivity of the detection kit according to example 6, in the embodiment with antibodies according to the invention vs. performed with antibodies from the commercial FebriDx assay. (Example 13)

### Examples

The invention will be further explained by way of examples with reference to the attached drawings.

### Example 1: Lysis and reaction buffer

Based on previous experimental work and to exclude mutual interaction and possible inhibition of the immunoreaction, we tested 35 variants of the lysis and reaction buffer, which contained Tris-HCl (0-100 mM), NaCl (0-1500 mM), BSA (0-3 % w/v), NaN3 (0-0.1 % w/v), CHAPS (0-2.5 % w/v) N,N-diethanolamide (0 or 1.2 % w/v), Block Ace (0-4 % w/v) a pH 6.5-9.5; and additional components (100 mM (4-(2-hydroxyethyl)-1-piperazineethanesulfonic acid (HEPES), 1 or 3 % w/v RPLA, 0.25 or 0.5 % w/v casein hydrosylate, 1x ELISA SynBlock, 20 % w/v Tween 20, 1 % w/v Brij L23). The composition of the tested buffers is in the table below.

| Buffer | Tris-HCl (mM) | NaCl (mM) | BSA (% w/v) | NaN₃ (% w/v) | CHAPS (% w/v) | N,N-Diethanolamide (% w/v) | Block Ace (% w/v) | pH | Additional component |
|---|---|---|---|---|---|---|---|---|---|
| 1 | 100 | 1500 | 0.1 | 0.1 | 2.5 | 1.2 | 4 | 8.5 | |
| 2 | 50 | 1500 | 0.1 | 0.1 | 2.5 | 1.2 | 4 | 8.5 | |
| 3 | 20 | 1500 | 0.1 | 0.1 | 2.5 | 1.2 | 4 | 8.5 | |
| 4 | 100 | 1000 | 0.1 | 0.1 | 2.5 | 1.2 | 4 | 8.5 | |
| 5 | 100 | 500 | 0.1 | 0.1 | 2.5 | 1.2 | 4 | 8.5 | |
| 6 | 100 | 150 | 0.1 | 0.1 | 5 | 1.2 | 4 | 8.5 | |
| 7 | 100 | 1500 | 0.1 | 0.1 | 1 | 1.2 | 4 | 8.5 | |
| 8 | 100 | 1500 | 0.1 | 0.1 | 0.5 | 1.2 | 4 | 8.5 | |
| 9 | 100 | 1500 | 0.1 | 0.1 | 2.5 | 1.2 | 4 | 8.5 | |
| 10 | 100 | 1500 | 0.1 | 0.1 | 2.5 | 1.2 | 2 | 8.5 | |
| 11 | 100 | 1500 | 0.1 | 0.1 | 2.5 | 1.2 | 1 | 8.5 | |
| 12 | 100 | 1500 | 0.1 | 0.1 | 2.5 | 1.2 | 4 | 6.5 | |
| 13 | 100 | 1500 | 0.1 | 0.1 | 2.5 | 1.2 | 4 | 7.5 | |
| 14 | 100 | 1500 | 0.1 | 0.1 | 2.5 | 1.2 | 4 | 9.5 | |
| 15 | 100 | 1500 | 0.1 | 0.1 | 2.5 | 1.2 | 4 | 8.5 | 100 m M HEPES |
| 16 | 100 | 1500 | 0.1 | 0.1 | 2.5 | 1.2 | 4 | 8.5 | 1 % w/v RPLA |
| 17 | 100 | 1500 | 0.1 | 0.1 | 2.5 | 1.2 | 4 | 8.5 | 0,25 % w/v Casein Hydrosylate |
| 18 | 100 | 1500 | 0.1 | 0.1 | 2.5 | 1.2 | 4 | 8.5 | 10 % w/v ELISA SynBlock |
| 19 | 100 | 1500 | 0.1 | 0.1 | 2.5 | 1.2 | 4 | 8.5 | 1 % w/v Tween 20 |
| 20 | 100 | 1500 | 0.1 | 0.1 | 2.5 | 1.2 | 4 | 8.5 | 1 % w/v Brij L23 |
| 21 | 0 | 1500 | 0.1 | 0.1 | 2.5 | 1.2 | 4 | 8.5 | 100 m M HEPES |
| 22 | 0 | 150 | 0.1 | 0.1 | 2.5 | 1.2 | 4 | 8.5 | 100 m M HEPES |
| 23 | 0 | 0 | 0 | 0 | 2.5 | 1.2 | 0 | 8.5 | 1x ELISA SynBlock |
| 24 | 0 | 0 | 0 | 0 | 0 | 0 | 0 | 8.5 | 1x ELISA SynBlock |
| 25 | 100 | 1500 | 0.1 | 0.1 | 2.5 | 1.2 | 0 | 8.5 | 3 % w/v RPLA |
| 26 | 100 | 150 | 0.1 | 0.1 | 2.5 | 1.2 | 0 | 8.5 | 3 % w/v RPLA |
| 27 | 100 | 1500 | 0.1 | 0.1 | 2.5 | 1.2 | 0 | 8.5 | 0.5 % w/v Casein Hydrosylate |
| 28 | 100 | 150 | 0.1 | 0.1 | 2.5 | 1.2 | 0 | 8.5 | 0,5 % w/v Casein Hydrosylate |
| 29 | 100 | 1500 | 3 | 0.1 | 2.5 | 1.2 | 0 | 8.5 | |
| 30 | 100 | 150 | 3 | 0.1 | 2.5 | 1.2 | 4 | 8.5 | |
| 31 | 100 | 1500 | 0.1 | 0.1 | 0 | 1.2 | 4 | 8.5 | 1 % w/v Tween 20 |
| 32 | 100 | 150 | 0.1 | 0.1 | 0 | 1.2 | 4 | 8.5 | 1 % w/v Tween 20 |
| 33 | 100 | 1500 | 0.1 | 0.1 | 0 | 1.2 | 4 | 8.5 | 1 % w/v Brij L23 |
| 34 | 100 | 150 | 0.1 | 0.1 | 0 | 1.2 | 4 | 8.5 | 1 % w/v Brij L23 |
| 35 | 100 | 1500 | 0.1 | 0.1 | 2.5 | 0 | 4 | 8.5 | |

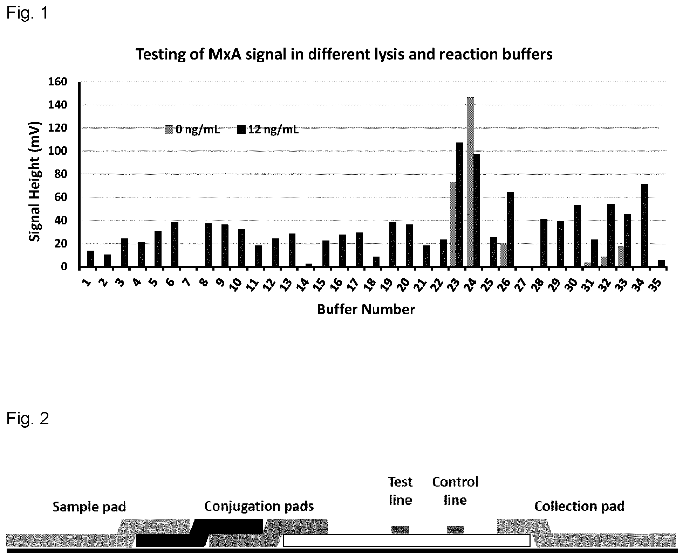

We measured the signal height of the test line at a concentration of purified native MxA protein of 0 or 12 ng/mL in all tested buffers. We added gold particle-conjugated mouse monoclonal antibody 1302.5.32 and biotin-conjugated mouse monoclonal antibody 1302.34.16 to the samples. Buffers containing MxA (or without MxA) and conjugated antibodies were incubated at room temperature for 5 min. We then applied 50 µL of sample to the LFT (the LFT contained an application pad, a detection zone with a test line containing streptavidin and a control line containing anti-mouse IgG, and a waste/collection pad). After 15 minutes, we quantified the signal on the test line using a reader. During the evaluation, we focused on the signal height and possible false positives of the test (Figure 1).

False positivity is observed especially with the use of ELISA SynBlock buffer, which is partially reduced by use of 2.5 % w/v CHAPS and N,N-diethanolamide. To a lesser extent, false positivity is observed with detergents other than CHAPS and/or low BSA levels. Lowering the NaCl concentration to 150 mM, and maintaining CHAPS at a concentration of 2.5% w/v and pH 8.5 led to a higher signal. The most advantageous variant is the buffer with the blocking agent BSA (3% w/v), which in this series of experiments provided a lower specific signal than the buffer with Block Ace. The exact composition of Block Ace is not published by the manufacturer.

To confirm the efficiency of the tested lysis and reaction buffers, we performed experiments using whole blood samples, in which the level of MxA mRNA expression was determined using quantitative PCR (1x negative sample, 2x positive sample). Buffers number 1, 6, 8, 26, 28, 30, 32 and 34 were selected for this experiment. Blood samples were diluted 10x in each of these buffers (ratio 1:9). The test itself was conducted identically to the execution described in this example above. The test results are in the table below.

| | **Test Line - Mean Signal Height (mV)** | | |
|---|---|---|---|
| | **MxA mRNA** | | |
| **Buffer** | **negative** | **positive** | **positive** |
| 1 | 0 | 0 | 0 |
| 6 | 0 | 0 | 22.2 |
| 8 | 0 | 0 | 0 |
| 26 | 0 | 45.9 | 68.6 |
| 28 | 0 | 36.8 | 57.6 |
| 30 | 0 | 45.2 | 78.2 |
| 32 | 5.6 | 0 | 10.3 |
| 34 | 0 | 0 | 15.8 |

The highest signals were obtained using lysis and reaction buffers 26, 28 and 30. These buffers have a lower concentration ofNaCl (150 mM instead of 1500 mM). There was no difference in the intensity of the control lines. Buffer 30 was used for further examples.

### Example 2: Preparation of conjugated antibodies and order of conjugation pads

Mouse monoclonal antibodies 1302.5.32 and 1302.34.16 are conjugated to biotin (sulfo-NHS-LC-LC-biotin) or detection particles using commercially available kits. If the conjugated antibodies are to be applied to the interaction zone pads, then the conjugated antibodies are applied to the pads where they are dried at pH 10 to ensure their stability. Each of the antibodies is applied to a separate pad so that their interaction does not occur during drying; the interaction could potentially lead to false positive test results and reducing the test's informative value.

To obtain the best combination of conjugated antibodies, the conjugation was performed in four variants - conjugation of antibody 1302.5.32 or 1302.34.16 with biotin (ratio 1:8) and conjugation of antibody 1302.5.32 or 1302.34.16 with gold particles. In the case of conjugated antibodies with gold particles, the coating concentration is 2 µg of antibody per OD (optical density) unit. pH levels of 7-10 (drying buffer) were tested for drying the conjugated antibodies on the pads. We observed aggregation of the conjugates deposited on the pads after drying, with aggregation occurring as a function of decreasing pH; drying at pH 10 did not result in aggregation of gold-conjugated antibody 1302.5.32, while gold-conjugated antibody 1302.34.16 tended to aggregate even at this pH.

To find the most suitable combination of conjugates, LFT was constructed in eight variants, which included different conjugations (1302.5.32-biotin/1302.34.16-gold or 1302.5.32-gold/1302.34.16-biotin), drying at pH 9 or 10 and the order pads on the test (SP-BCP-DCP or SP-DCP-BCP; SP - sample pad, DCP - detection particles conjugate pad, BCP - biotin conjugate pad). We tested samples of reaction and lysis buffer containing 0 and 12 ng/mL of purified native human MxA (50 µL of sample loaded, 15 min incubation time) and the results were quantified using a reader as signal level (mV) (see table below).

| | Test Line - Mean Signal Height (mV) | |
|---|---|---|
| LFT Setup | 0 ng/mL MxA | 12 ng/mL MxA |
| 1302.5.32-biotin/1302.34.16-gold/pH 9/SP-DCP-BCP | 0 | 106 |
| 1302.5.32-biotin/1302.34.16-gold/pH 9/SP-BCP-DCP | 0 | 137 |
| 1302.5.32-biotin/1302.34.16-gold/pH 10/SP-DCP-BCP | 0 | 188 |
| 1302.5.32-biotin/1302.34.16-gold/pH 10/SP-BCP-DCP | 0 | 214 |
| 1302.5.32-gold/1302.34.16-biotin/pH 9/SP-DCP-BCP | 0 | 295 |
| 1302.5.32-gold/1302.34.16-biotin/pH 9/SP-BCP-DCP | 0 | 357 |
| 1302.5.32-gold/1302.34.16-biotin/pH 10/SP-DCP-BCP | 0 | 345 |
| 1302.5.32-gold/1302.34.16-biotin/pH 10/SP-BCP-DCP | 0 | 298 |

The combination 1302.5.32-gold/1302.34.16-biotin provides a stronger signal than the combination 1302.5.32-biotin/1302.34.16-gold. One reason is the tendency of the 1302.34.16-gold conjugate to aggregate. This aggregation can be prevented by sonicating the conjugate immediately before applying and drying the conjugate on the conjugation pad. Drying conjugated antibodies at pH 10 results in higher assay sensitivity than at pH 9 for all assay variants. The SP-DCP-BCP pad sequence provides higher assay sensitivity for most assay variants.

To evaluate the most favourable detection limit for different combinations of conjugated antibodies, we tested the above-mentioned conjugations (1302.5.32-biotin/1302.34.16-gold or 1302.5.32-gold/1302.34.16-biotin) with antibodies drying at pH 10 and with the order of the pads SP-DCP-BCP. The tested samples were either lysis and reaction buffer or lysed whole blood (diluted 10x in lysis and reaction buffer) with the addition of purified native MxA protein (0/1.2/3 ng). 50 µL of the sample was applied to the sample pad and the incubation time was 15 minutes. The test results are in the table below.

| | **Mean Signal Height (mV)** | | | |
|---|---|---|---|---|
| | **Buffer** | | **Whole Blood** | |
| **MxA (ng/mL)** | **1302.5.32-gold/1302.34.16-biotin** | **1302.5.32-biotin/1302.34.16-gold** | **1302.5.32-gold/1302.34.16-biotin** | **1302.5.32-biotin/1302.34.16-gold** |
| **0** | 0 | 5 | 0 | 0 |
| **1.2** | 8 | 27 | 0 | 33 |
| **3** | 52 | 73 | 67 | 98 |

The more preferred combination is the conjugation of antibody 1302.5.32 with biotin and antibody 1302.34.16 with gold particles with better signal height at a near cut-off for 10x diluted MxA concentration of 1.2 ng/mL both in buffer and whole blood. The calculated detection limit for the combination 1302.5.32-biotin/1302.34.16-gold is 2.5 times lower than that for the combination 1302.5.32-gold/1302.34.16-biotin. The 1302.5.32-biotin/1302.34.16-gold combination is therefore more sensitive despite the tendency of the 1302.34.16-gold conjugate to aggregate.

### Example 3: Antibody quantities on pads

To ensure the optimal concentration of antibodies, we prepared several variants of the test with conjugated antibodies applied and dried on the pads and placed in the interaction zone of the test. We applied biotin-conjugated mouse antibody 1302.5.32 to the pad at 0.1, 0.2, 0.4 and 0.6 µg/LFT and gold particle-conjugated mouse antibody 1302.34.16 at 50, 65 and 80 mOD. We tested samples with purified native human MxA at concentrations of 0 and 3 ng/mL in the lysis and reaction buffer when applying 50 µL of the sample. The results of titration of the concentration of conjugated antibodies are in the table below.

| | Test Line | | Control Line | |
|---|---|---|---|---|
| Gold/biotin conjugates concentration | Mean Signal Height at 0 ng/mL MxA (mV) | Mean Signal Height at 3 ng/mL MxA (mV) | Mean Signal Height at 0 ng/mL MxA (mV) | Mean Signal Height at 3 ng/mL MxA (mV) |
| 50 mOD/0.1 µg | 0 | 146 | 931 | 939 |
| 50 mOD/0.2 µg | 0 | 168 | 941 | 935 |
| 50 mOD/0.4 µg | 1 | 133 | 957 | 984 |
| 50 mOD/0.6 µg | 0 | 107 | 932 | 932 |
| 65 mOD/0.1 µg | 0 | 181 | 944 | 925 |
| 65 mOD/0.2 µg | 0 | 180 | 915 | 958 |
| 65 mOD/0.4 µg | 5 | 173 | 944 | 981 |
| 65 mOD/0.6 µg | 0 | 121 | 991 | 964 |
| 80 mOD/0.1 µg | 2 | 182 | 968 | 987 |
| 80 mOD/0.2 µg | 0 | 200 | 947 | 983 |
| 80 mOD/0.4 µg | 0 | 159 | 943 | 974 |
| 80 mOD/0.6 µg | 0 | 148 | 953 | 964 |

The false positive signal was very low or absent. Higher concentrations of antibody conjugated to gold particles generally resulted in a higher signal on the test line. The maximum signal was obtained at a concentration of 0.2 µg of biotinylated antibody for all concentrations of the antibody conjugated with gold particles. Higher concentrations of antibody conjugated to gold particles however result in a visible background that is enhanced when using blood samples. Therefore, the most advantageous alternative is to combine the biotin-conjugated anti-MxA mouse antibody 1302.5.32 at 0.2 µg/LFT with the gold particle-conjugated mouse antibody 1302.34.16 at 50 mOD/LFT (0.1 µg/LFT).

### Example 4: Detection zone

It was necessary to adjust the amount of streptavidin applied to the membrane in the detection zone to achieve the appropriate quantification of the result. The detection zone was prepared with the application of a control strip (anti-mouse IgG) with a standard concentration of 1 mg/mL. After a series of experiments and narrowing the range of usable concentrations, three concentrations of applied streptavidin (SA) were used for the final experiment - 0.8, 1.0 and 1.2 mg/mL with a spraying amount of 0.75 µL/cm.

We tested samples with purified native human MxA at concentrations of 0 and 12 ng/mL in lysis and reaction buffer (50 µL of sample loaded, 15 min incubation time) and the results were quantified using a reader (see table below).

| | Test Line | | Control Line | |
|---|---|---|---|---|
| | Mean 0 ng/mL MxA (mV) | Mean 12 ng/mL MxA (mV) | Mean 0 ng/mL MxA (mV) | Mean 12 ng/mL MxA (mV) |
| 0.8 mg/mL SA | 0 | 179 | 1559 | 1553 |
| 1.0 mg/mL SA | 0 | 222 | 1541 | 1574 |
| 1.2 mg/mL SA | 0 | 234 | 1547 | 1542 |

Higher concentrations of streptavidin result in a higher signal on the test line. We reached almost the saturation level, from which there was no longer a significant increase in signal between concentrations of 1.0 and 1.2 mg/mL of streptavidin. The most favourable concentration of streptavidin was determined to be 1.2 mg/mL.

### Example 5: Construction of a detection kit

The first embodiment is a kit for the detection of human MxA protein, which includes a mouse monoclonal antibody 1302.5.32 against human protein MxA conjugated with biotin or gold particles and a mouse monoclonal antibody 1302.34.16 against human protein MxA conjugated with biotin or gold particles. In this embodiment, a combination of biotin-conjugated anti-human MxA mouse monoclonal antibody 1302.5.32 and gold-conjugated anti-human MxA mouse monoclonal antibody 1302.34.16 is more preferred when analysing samples with 0 ng/mL or 12 ng/mL of purified native human MxA in lysis and reaction buffer. In this embodiment, this combination provided a higher signal (see table below). Each antibody is in a separate container, or in a separate compartment of a container. The antibodies are dissolved in the lysis and reaction buffer. Furthermore, the kit contains a detection system in the form of a membrane based on nitrocellulose or cellulose acetate with an application zone for the application of the tested sample, a detection zone with streptavidin, a control zone with anti-mouse IgG and a collection waste zone. Zones follow each other in the order listed. The set also contains a lysis and reaction buffer with a composition of 100 mM TRIS-HCl, 150 mM NaCl, 0.1% w/v BSA, 0.1% w/v NaN3, 2.5% w/v CHAPS, 1.2% w/v N,N-diethanolamide and 3% w/v BSA.

| | Test Line - Mean Signal Height (mV) | |
|---|---|---|
| LFT Setup | 0 ng/mL MxA | 12 ng/mL MxA |
| 1302.5.32-biotin/1302.34.16-gold | 0 | 600 |
| 1302.5.32-gold/1302.34.16-biotin | 0 | 320 |

### Example 6: Use of the detection kit

This example describes the performing of the test and the function of the kit described in Example 5. Before applying the tested sample, e.g. whole blood cell lysate, the sample is diluted 1:9 with lysis and reaction buffer, mixed and incubated with conjugated antibodies 1302.5.32 and 1302.34.16, and then applied to the application zone of the development device. In the case of a positive reaction, a sandwich immunocomplex of the MxA protein and the antibodies is formed during incubation. Antibody 1302.5.32 targets the C-terminal epitope of the human MxA protein, particularly positions 430-662, and antibody 1302.34.16 targets the N-terminal epitope of the human MxA protein, particularly positions 1-429. From the application zone, all components subsequently migrate to the detection zone, where both unreacted 1302.5.32 antibodies conjugated with biotin and the sandwich immunocomplex via the biotin-streptavidin bond are captured. In the detection zone, this immunocomplex is detected via antibody 1302.34.16 conjugated with gold particles visually, photometrically or densitometrically in the visible spectrum. Unreacted antibodies 1302.34.16 conjugated with gold particles also migrate from the application zone, but they are not captured in the detection zone, but only in the control zone through the binding of mouse antibody and anti-mouse IgG. In the control zone, these 1302.34.16 antibodies are detected visually, photometrically or densitometrically in the visible spectrum, and the detection in the control zone serves as a control of the entire test. All other unreacted migrating components are finally captured in the waste collection zone.

### Example 7: Construction of a detection kit

Another exemplary embodiment is the human MxA protein detection kit using the development device described in Example 5. This kit uses the same pair of monoclonal antibodies 1302.5.32 and 1302.34.16 against the human MxA protein, but in this case the monoclonal antibody 1302.5.32 is conjugated to gold particles and the monoclonal antibody 1302.34.16 is conjugated with biotin. Each antibody is in a separate container or in a separate compartment in a container, the antibodies are in the lysis and reaction buffer, which preferably has the composition described in Example 2. The detection kit also includes a container with lysis and reaction buffer.

### Example 8: Use of the detection kit

This example describes the performing of the test and the function of the kit described in Example 7. Before applying the tested sample, e.g. whole blood cell lysate, the sample is diluted 1:9 (a 1:5 dilution was also tested, which leads to a slightly lower sensitivity due to higher background) with lysis and reaction buffer, mixed and incubated with conjugated antibodies 1302.5.32 and 1302.34.16, and then applied to the application zone of the detection system. In the case of a positive reaction, a sandwich immunocomplex of the MxA protein and the antibodies is formed during incubation. Antibody 1302.5.32 targets the C-terminal epitope of the human MxA protein, particularly positions 430-662, and antibody 1302.34.16 targets the N-terminal epitope of the human MxA protein, particularly positions 1-429. From the application zone, all components subsequently migrate to the detection zone, where both unreacted 1302.34.16 antibodies conjugated with biotin and the sandwich immunocomplex via the biotin-streptavidin bond are captured. In the detection zone, this immunocomplex is detected via antibody 1302.5.32 conjugated with gold particles visually, photometrically or densitometrically in the visible spectrum. Unreacted antibodies 1302.5.32 conjugated with gold particles also migrate from the application zone, but they are not captured in the detection zone, but only in the control zone through the binding of mouse antibody and anti-mouse IgG. In the control zone, these 1302.5.32 conjugated antibodies are detected visually, photometrically or densitometrically in the visible spectrum, and the detection in the control zone serves as a control of the entire test. All other unreacted migrating components are finally captured in the waste collection zone.

### Example 9: Construction of a detection kit

The third, most preferred embodiment described in these examples is a kit for the detection of human MxA protein, the development device of which is shown in Fig. 2. This kit includes a development device containing an application zone for the application of the tested sample, an interaction zone including separate spaces (pads) for each of the pair of conjugated monoclonal antibodies 1302.5.32 (conjugated with biotin) and 1302.34.16 (conjugated with gold particles) against the human MxA protein. The antibodies are immobilized on separate pads as shown in Example 1. A gold-conjugated antibody is applied closer to the application zone, followed by a biotin-conjugated antibody, to increase the solubility and effectiveness of the gold conjugate. The gold-conjugated antibody can be sonicated immediately before being applied to the pad, in order to reduce aggregation. The amount of mouse monoclonal antibody 1302.34.16 conjugated to gold particles is 50 mOD. The amount of mouse monoclonal antibody 1302.5.32 conjugated to biotin using a biotin ligand is 0,15 -0.2 µg. The development device also has a detection zone with streptavidin, a control zone with anti-mouse IgG and a collection waste zone. The development device is in the form of a strip based on nitrocellulose. The detection kit also contains a lysis and reaction buffer with a composition of 100 mM TRIS-HCl, 150 mM NaCl, 0.1% w/v BSA, 0.1% w/v NaN₃, 2.5% w/v CHAPS, 1.2 % w/v N,N-diethanolamide and 3% w/v BSA. The lysis and reaction buffer has a pH of 8.5.

### Example 10: Use of the detection kit

This example describes the use of the detection kit according to example 9. In the case of this detection kit, a sample of whole blood, e.g. capillary blood, (5-10 µL, more preferably 5 µL) is applied to the application zone and then lysis and reaction buffer (50-100 µL, more preferably 50 µL) is applied 2-11 minutes later. Thus, cell lysis occurs directly in the application zone of the development device of the kit for the detection of the human MxA protein.

From the application zone, all components subsequently migrate to the interaction zone, where conjugated antibodies 1302.5.32 and 1302.34.16 are immobilized on separate pads, when the concentration of mouse monoclonal antibody 1302.34.16 conjugated with gold particles is 50 mOD and the concentration of mouse monoclonal antibody 1302.5.32 conjugated with biotin is 0.15-0.2 µg.

In the case of a positive reaction, a sandwich immunocomplex of MxA protein and the conjugated antibodies is formed in the interaction zone. Antibody 1302.5.32 targets the C-terminal epitope of the human MxA protein, particularly positions 430-662, and antibody 1302.34.16 targets the N-terminal epitope of the human MxA protein, particularly positions 1-429. Further, the components, including the resulting sandwich immunocomplex, migrate to the detection zone, where both unreacted 1302.5.32 antibodies conjugated with biotin and the sandwich immunocomplex are captured through the biotin-streptavidin bond. In the detection zone, this immunocomplex is detected via antibody 1302.34.16 conjugated with gold particles visually, photometrically or densitometrically in the visible spectrum. Unreacted antibodies 1302.34.16 conjugated with gold particles also migrate from the application zone, but they are not captured in the detection zone, but only in the control zone through the binding of mouse antibody and anti-mouse IgG. In the control zone, these 1302.34.16 antibodies are detected visually, photometrically or densitometrically in the visible spectrum, and the detection in the control zone serves as a control of the entire test. All other unreacted migrating components are finally captured in the waste collection zone.

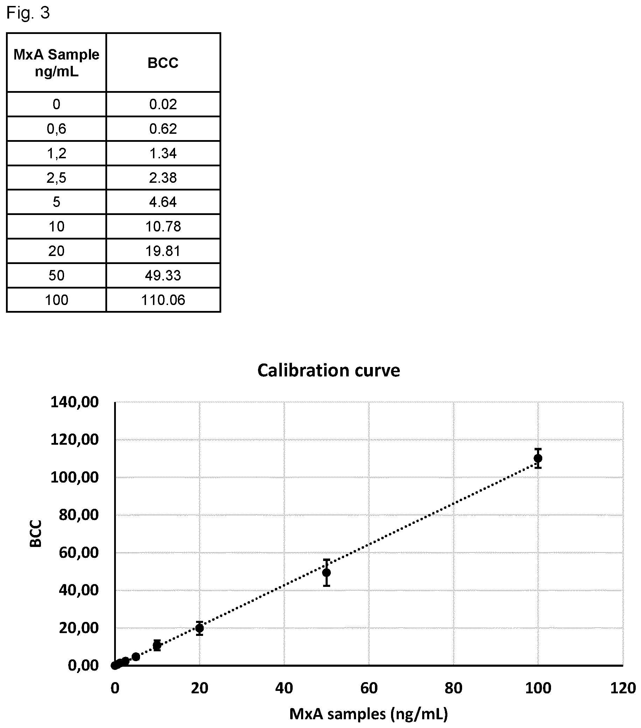

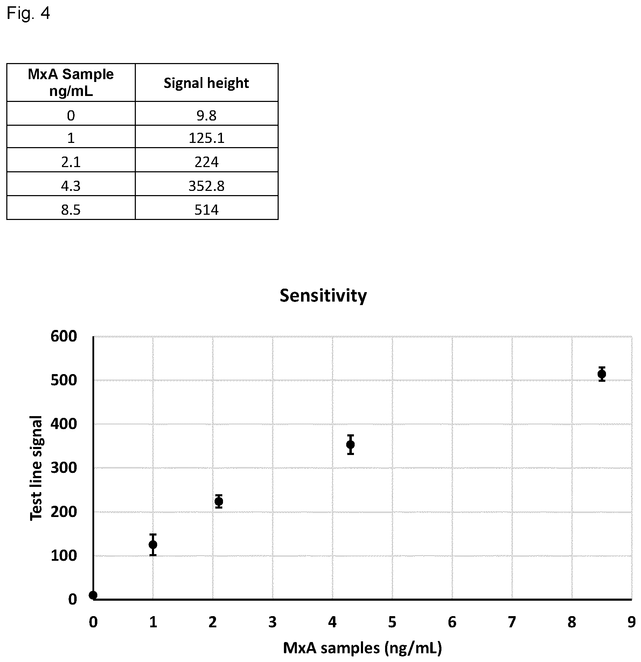

With this embodiment, the detection result is obtained within 10 to 15 minutes. Fig. 3 shows an example of a calibration curve measured using the procedure described here. Fig. 4 shows the results of the sensitivity test of the mentioned detection kit, the calculated limit of detection is 0.3 ng/mL MxA protein.

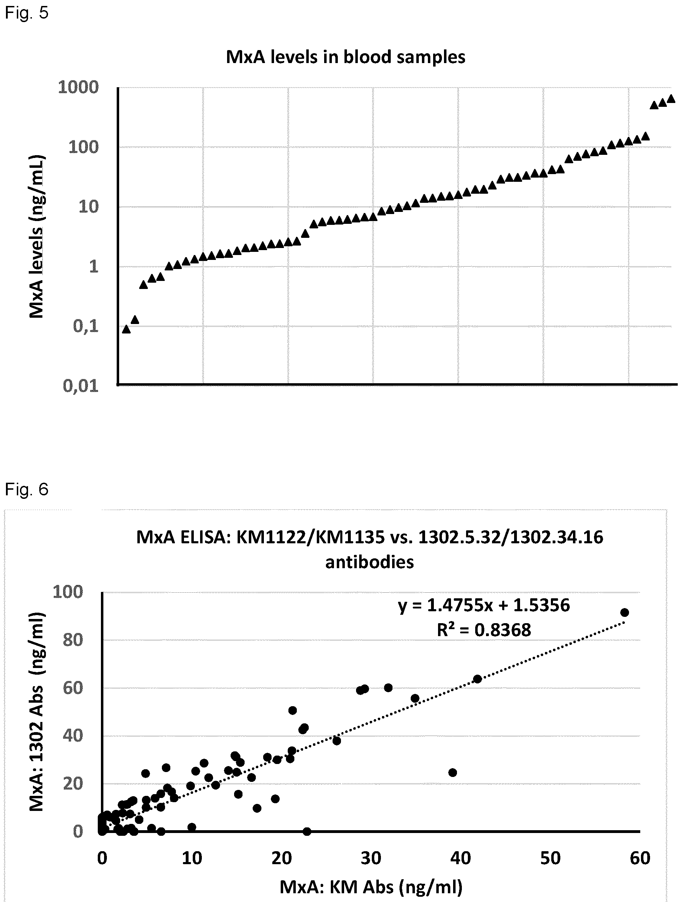

Fig. 5 shows the detection results for 65 whole blood samples.

Alternatively, and even more advantageously, it is possible to lyse whole blood cells before applying the tested sample onto the application zone, e.g. for whole blood cell lysate, the sample is diluted 1:5-1: 10 (preferably 1:9) with lysis and reaction buffer, and then the lysate is directly applied to the application zone of the development device (50 µL).

In both cases, it is preferable to wash the membrane with lysis and reaction buffer (50-100 µl, more preferably 50 µL) 2-11 minutes after sample application (more preferably 10-11 minutes). This step significantly reduces the background caused by released hemoglobin from lysed erythrocytes.

With these embodiments, the detection result is obtained within 10 to 15 minutes. Fig. 3 shows an example of a calibration curve measured using the procedure described here. Fig. 4 shows the results of the sensitivity test of the mentioned detection kit, the calculated limit of detection is 0.3 ng/mL MxA protein.

Fig. 5 shows the detection results for 65 whole blood samples.

### Example 11: Comparison of the LFT according to the invention and a commercially available LFT

In this example, the LFT assay prepared according to Example 9 is compared to a commercially available FebriDx^{®} LFT assay. The FebriDx^{®} assay uses antibodies KM1124 and KM1135 conjugated to biotin and gold particles. According to the information available, the lysis buffer in the FebriDx^{®} test has the following composition (in % w/v): 100 mM HEPES buffer (pH 8.0) containing 5% CHAPS and 2% NP-40 with 150 mM sodium chloride, 0.1% BSA, and 0.1% sodium azide.

In the first experiment, a sample of non-clotting whole blood containing 62.5 ng/mL of MxA protein was tested. 5 µL of sample (volume as recommended by the manufacturer) was applied onto the FebriDx^{®} test and after the period of 10 minutes, as recommended by the manufacturer, a control line was clearly visible, but no signal was visible in the test line, i.e., the presence of MxA was not detected.

Subsequently, purified native human MxA protein with a concentration of 100 ng/mL in FebriDx^{®} lysis buffer was tested. 5 µL of the sample was applied onto the FebriDx^{®} assay and after 10 minutes, a control strip was clearly visible, but still no signal was visible in the test line, i.e., the presence of MxA was not detected.

Subsequently, the same sample at volume of 25 µL (five times the volume recommended by the manufacturer) was applied onto the FebriDx^{®} test and after 10 minutes, both the control and MxA detection lines were clearly visible in the detection zone.

The test according to the present invention was used in another experiment for testing when applying 5 µL of the purified native human MxA protein in the lysis and reaction buffer and subsequent application of the buffer. Tested concentrations of the human MxA were 0, 1, 2.1, 4.3 and 8.5 ng/mL. After 10 minutes, the test was evaluated. In the absence of MxA, the control line was clearly visible and no signal was visible in the test line. At the MxA concentration of 1 ng/mL, the control line was clearly visible and a faint band was visible in the test line. At the MxA concentration of 2.1 ng/mL, the control line was clearly visible and the test line was visible. At the MxA concentration of 4.3 ng/mL, the control line was clearly visible and the test line was clearly visible. At the MxA concentration of 8.5 ng/mL, the control line was clearly visible, and there was a clearly visible test line that was comparable in intensity to the control line.

### Example 12: Comparison of sensitivity of the antibodies according to the invention and the antibodies used in the FebriDx^{®} test

We compared the sensitivity of the pair of mouse monoclonal MxA antibodies 1302.34.16 and 1302.5.32 (antibodies 1302) used in this invention with the mouse monoclonal MxA antibodies KM1124 and KM1135 (antibodies KM), which are used in the commercially available FebriDx^{®} test kit. The measurement was performed on 158 blood samples using the MxA Protein Human ELISA kit (Biovendor - Laboratorni medicína a.s.) according to the manufacturer's instructions. Antibodies 1302 showed 1.55x higher sensitivity than KM antibodies (Fig. 5). The correlation of the antibody potency expressed as R² was 0.84. Low MxA values (< 10 ng/mL in blood samples that were evaluated as zero according to KM analysis) were also measured with 1302 antibodies.

The result of the comparison is shown in Fig. 6.

### Example 13: Comparison of the development device of the invention in combination with the antibodies of the invention vs. in combination with the antibodies from a commercial assay

A development system was prepared according to Example 9, except that the KM1124 and KM1135 antibodies used in the commercial FebriDx^{®} LFT test were used for the preparation.

For comparison, a development device prepared according to Example 9 was used (mouse monoclonal antibody 1302.34.16 conjugated with gold particles and mouse monoclonal antibody 1302.5.32 conjugated with biotin).

Thus, the comparison was performed with the same development device, only the antibodies differed. The development device was prepared with KM antibodies in two ways: in one embodiment, the antibody KM1124 was conjugated with gold particles and the antibody KM1135 with biotin, and in the second, the antibody KM1135 was conjugated with gold particles and the antibody KM1124 with biotin. MxA-negative whole blood samples supplemented with native human MxA protein at MxA concentrations of 1, 5, or 10 ng/mL were used as tested samples. The tested samples were lysed in a 1:9 ratio in lysis and reaction buffer. Lysed samples were applied in an amount of 50 µL, and after 10 minutes, lysis and reaction buffer in an amount of 50 microliters was applied for washing. Quantitative assessment was carried out using a reader.

The results are described in the table below.

| | LFT with antibodies 1302.34.16 with gold and 1302.5.32 with biotin (Mean Signal Height; mV) | LFT with antibodies from the commercial test (Mean Signal Height; mV) | |
|---|---|---|---|
| MxA (ng/mL) | | KM1124 with gold | KM 1135 with gold |
| | | KM1135 with biotin | KM1124 with biotin |
| 0 | 0.008 | 0.013 | 0.026 |
| | | | |
| 1 | 0.06 | 0.025 | 0.04 |
| 5 | 0.205 | 0.07 | 0.078 |
| 10 | 0.38 | 0.11 | 0.097 |

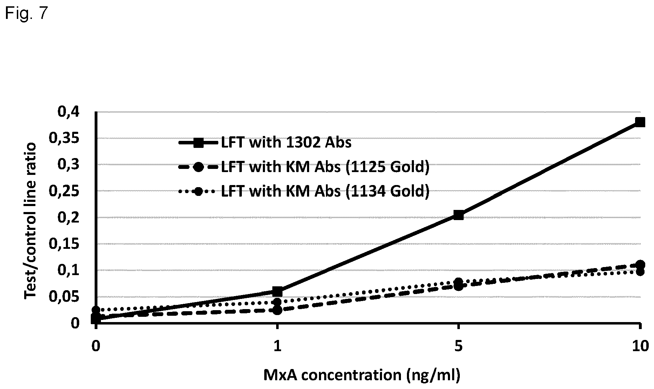

The results are also shown in Fig. 7. The results show that the antibody sensitivity of the commercial FebriDx^{®} test is improved with the development device of the invention. However, it still fails to achieve the sensitivity of the antibodies of the invention in combination with this development device. It can therefore be concluded that the antibodies according to the invention have a higher sensitivity than the antibodies from the commercial test, and further that the design of the development device and the buffer further improve the sensitivity of the antibodies, while acting synergistically with the antibodies according to the invention.

### Example 14: Testing of samples from patients with acute respiratory infection

The detection kit prepared according to Example 9 was used to test capillary blood samples from patients with acute respiratory infection. The sample assaying procedure was as described in Example 10, but the tested samples were applied in the form of lysates.

The following table shows the correct diagnosis of a viral or bacterial pathogen (determined from an oroor nasopharyngeal swab) in comparison with the result of the test for the presence of MxA performed according to the invention from a capillary blood sample. This confirms the clinical relevance of the kit and method of the invention.

| | | Patients | MxA pos. | MxA neg. |
|---|---|---|---|---|
| Influenza A | | 18 | 17 | 1 |
| Influenza B | | 8 | 8 | 0 |
| SARS-CoV-2 | | 10 | 10 | 0 |
| Human adenovirus | | 3 | 3 | 0 |
| Metapneumovirus | | 2 | 2 | 0 |
| Human respiratory syncytial virus | | 2 | 2 | 0 |
| Coronavirus (other than SARS) | | 2 | 2 | 0 |
| Enterovirus | | 2 | 2 | 0 |
| Rhinovirus | | 1 | 1 | 0 |
| Parainfluenza virus | | 1 | 1 | 0 |
| virus not detected | bacteria - | 1 | 1 | 0 |
| | bacteria + | 28 | 0 | 28 |
| Total | | 78 | 49 | 29 |

The next table summarizes the sensitivity, specificity and other parameters of the detection kit according to the invention:

| | MxA levels | |
|---|---|---|
| Virus detected | positive | negative |
| YES | 48 | 1 |
| NO | 1 | 28 |
| Sensitivity (%) | 98,0 | |
| Specificity (%) | 96,6 | |
| Positive predictive value | 1,0 | |
| Negative predictive value | 1,0 | |
| Likelihood ratio (pos.) | 28,4 | |
| Likelihood ratio (neg.) | 0,0 | |

### Industrial Applicabilitv

The present invention can be used in detecting the level of human MxA protein in tested samples, e.g. directly in whole blood samples or whole blood cell lysate samples. This can be used in differential diagnosis of infectious diseases, reducing incorrect prescriptions of antibiotic treatment and the overuse of antibiotics. A further use of the present invention besides diagnostics is the monitoring of the treatment of viral or autoimmune or oncological diseases, which are characterized by a condition induced by interferons of types I and III.

## Claims

1. A pair of mouse monoclonal anti-human MxA protein antibodies, clones 1302.5.32 a 1302.34.16, wherein one of the antibodies is conjugated with biotin and the other of the antibodies is conjugated with detection particles selected from gold particles, dyed latex particles and cellulose nanospheres, wherein the detection particles are preferably gold particles.

2. The pair of antibodies according to claim 1, wherein the antibody conjugated with biotin is obtainable by biotinylation wherein the ratio of the antibody to a biotinylation agent is within the range of 1:8 to 1:33; wherein the biotinylation agent is preferably sulpho-N-hydroxysuccinimide-aminocaproyl-aminocaproyl-biotin.

3. A lateral flow test kit for detection of human MxA protein in a tested sample, the kit comprising the pair of antibodies according to claim 1 or 2, lysis and reaction buffer, and a development device with an application zone for the application of the tested sample, a detection zone with a ligand of biotin and a control zone with anti-mouse antibody, wherein a membrane of the development device is based on nitrocellulose or cellulose acetate.

4. The kit according to claim 3, wherein the mouse monoclonal antibody 1302.5.32 is conjugated with biotin and the mouse monoclonal antibody 1302.34.16 is conjugated with detection particles.

5. The lateral flow test kit according to claim 3, wherein the the development device contains an interaction zone including a first pad on which a first conjugated mouse monoclonal antibody from the pair of conjugated mouse monoclonal antibodies according to claim 1 or 2 is immobilized and a second pad on which a second conjugated mouse monoclonal antibody from the pair of conjugated mouse monoclonal antibodies according to claim 1 or 2 is immobilized, wherein the interaction zone is located between the application zone and the detection zone.

6. The kit according to claim 5, wherein the application zone contains a sample pad (SP), and the interaction zone contains a biotin conjugate pad (BCP) containing the antibody conjugated with biotin and a detection conjugate pad (DCP) containing the antibody conjugated with detection particles, and wherein preferably the pads are arranged in the order SP-DCP-BCP.

7. The kit according to any one of claims 3 to 6, wherein the ligand of biotin is streptavidin and/or the anti-mouse antibody is anti-mouse IgG.

8. The kit according to any one of claims 3 to 7, wherein the lysis and reaction buffer contains the following components:
- Tris-HCl in an amount within the range 75-125 mM
- NaCl or KCl in an amount within the range 100-1500 mM
- NaN₃ in an amount within the range 0,08-1.5% w/v
- CHAPS in an amount within the range 2-3% w/v
- N,N-Diethanolamid in an amount within the range 1-1.5% w/v
- 2-3% w/v BSA and/or 0.3-1% w/v casein hydrolysate and/or 1-4% w/v RPLA1.
and has a pH within the range 7 to 9.

9. A method for detecting human MxA protein in a tested sample using the kit according to any one of claims 3 to 8, the method comprising the following steps:
a. lysis of capillary or venous whole blood sample in lysis and reaction buffer;
b. forming a sandwich immunocomplex of the human MxA protein, biotin-conjugated mouse monoclonal antibody, and detection particle-conjugated mouse monoclonal antibody, if human MxA protein is present in the tested sample;
c. capturing the sandwich immunocomplex in the detection zone via binding of the biotin-conjugated mouse monoclonal antibody to the biotin ligand, if human MxA protein is present in the tested sample;
d. detecting the sandwich immunocomplex in the detection zone by means of a mouse monoclonal antibody conjugated to the detection particles in the detection zone, preferably visually, photometrically or densitometrically in the visible light spectrum, if human MxA protein is present in the tested sample; and
e. detecting the mouse monoclonal antibody conjugated with detection particles in the control zone, preferably visually, photometrically or densitometrically in the visible light spectrum.

10. The method according to claim 9, wherein the kit according to claim 3 or 4 is used, and the sandwich immunocomplex is obtained by adding both conjugated mouse monoclonal antibodies to the lysed tested sample and by their reaction with the human MxA protein contained in the tested sample; wherein the lysed sample and the conjugated antibodies are dissolved in lysis and reaction buffer.

11. The method according to claim 9, wherein the kit according to claim 5 or 6 is used, and the sandwich immunocomplex is obtained by applying the lysed tested sample onto the application zone of the development device, and by subsequent reaction of the conjugated mouse monoclonal antibodies with the human MxA protein contained in the tested sample in the interaction zone of the development device.

12. The method according to any one of claims 9 to 11, wherein after 2-11 minutes from the application of the lysed tested sample and optionally of the antibodies onto the application zone of the development device, a further amount of lysis and reaction buffer is added.

13. In vitro use of the pair of mouse monoclonal antibodies according to claim 1 or 2, or of the kit according to any one of claims 3 to 8, for detection of human MxA protein, and/or for detection of viral or autoimmunity diseases, and/or for monitoring of the treatment of viral or autoimmunity or oncological diseases using interferon α and/or β, and/or for classification of obscure infections in oncological patients.
